# EUROPEAN PATENT SPECIFICATION

(11) **EP 0 368 343 B1**
(45) Date of publication and mention of the grant of the patent: **30.06.1993**
(21) Application number: 89120897.7
(22) Date of filing: 10.11.1989
(51) Int. Cl.: A61K 31/44

(54) **Ameliorating composition for dysmnesia**
Dysmnesie modifizierende Arzneimittel
Composition modifiant la dysmnésie

(30) Priority: 11.11.1988 JP 285217/88
(43) Date of publication of application: 16.05.1990
(73) Proprietor: KYORIN SEIYAKU KABUSHIKI KAISHA, Chiyoda-ku Tokyo (JP)
(72) Inventor: Ohashi, Mitsuo, Omiya-shi Saitama-ken (JP); Taga, Fukuraro, Minamisaitama-gun Saitama-ken (JP); Hirayama, Takashi, Kitakatsushika-gun Saitama-ken (JP)
(74) Representative: VOSSIUS & PARTNER

(56) References cited:
- GB-A- 1 378 375
- US-A- 4 647 566
- ARCH. INT. PHARMACODYN., vol. 280, 1986; M. OHASHI et al., pp. 216-229#
- JAPAN PHARMACOL. THER., vol. 13, no. 11, November 1985; Y. KUDO et al., pp. 217-221#
- FOLIA PHARMACOL. JAPAN, vol. 85, 1985; Y. KUDO et al., pp. 435-441#
- ANNALS NEW YORK ACADEMY SCIENCE, vol. 444, 1985; B. SALETU et al., pp. 406-427#
- JAPAN J. PHARMACOL. 1989, vol. 49 (suppl.), 62nd General Meeting of the Japanese Pharmacol. Soc., 25-28 March 1989; A. YAMANISHI et al., no. P296#

## Description

The present invention relates to an ameliorating composition for dysmnesia (impairment of memory), particularly to an ameliorating composition for dysmnesia containing a derivative of pyrazolo[1,5-a]pyridine as an active ingredient.

Recently diseases such as senile dementia accompanying dysmnesia have become a great medical and social problem as a result of prolonged human life expectancy.

Dysmnesia patients show a loss of intellectual ability, dysmnesia (namely impairment of memory), impairment of abstract thinking, aphasia, apraxia and disorientation. Such impairment of fundamental function results from a disorder of formation of memory or of reproduction of the memory retained. However, no effective medicament therefor has been found at the moment, so that early development of a medicament for treatment is very desirable.

The following pyrazolo[1,5-a] pyridine derivatives, found by Irikura et al.,are known (Japanese Patent Publication No. 52-29318 corresponding to U.S. Patent 3,850,941, U.K. Patent 1,378,375 and so on) to be effective for the treatment of circulatory organ disease, for the prevention and treatment of bronchial asthma, for an antiallergic action for ophthalmic diseases, and for an antirheumatic action, and still further be useful for the treatment of cerebrovascular disease. They may be represented by the following formula: 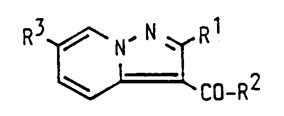
wherein R¹ and R² are independently hydrogen or an alkyl of 1 - 4 carbons, R³ is hydrogen or an alkyl of 1 - 3 carbons or an alkoxy of 1 - 3 carbons.

Folia Pharmacol. Japan., 85, 435-441 (1985), Kudo et al., discloses that 3-isobutyryl-2-isopropylpyrazolo(1,5-a)pyridine (KC-404) exerts a favourable influence on cerebral metabolism of rats with experimentally caused cerebral infraction. See the summary, 1) on p. 441. In particular, the compound increases the cerebral ATP.

Arch. int. Pharmacodyn., 280, pp. 216-229, (1986), Ohashi et al., teaches that KC-404 possesses a potent and selective cerebral vasodilating activity (page 217).

Kudo et al., Japan Pharmacol. Ther. 1985, Vol. 13, No. 11, pp. 217-221, teaches that KC-404 increases the partial oxygen pressure in cerebral tissue, thus suggesting a favourable effect on hypoxia.

It appears that the pharmacological activities reported above have been recognised to be correlated to mnestic troubles as shown by Ann. N.Y. Acad. Sci. 1985, Vol. 444, pp. 406-427, particularly by the passage of pages 416 to 420, of page 422, lines 5-7, and of page 424, lines 2 to 9.

Such pyrazolo[1,5-a]pyridine derivatives, however, were not known to be effective in ameliorating dysmnesia until now.

After extensive research it was found that the pyrazolo[1,5-a]pyridine derivatives which are known to be effective for the treatment of circulatory organ disease have a remarkable ameliorating effect on dysmnesia.

The object of the present invention is to provide an ameliorating composition for dysmnesia.

The present invention provides an ameliorating composition for dysmnesia, comprising as an active ingredient at least one pyrazolo[1,5-a]pyridine derivative represented by the general formula: 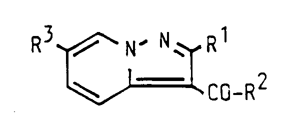
wherein R¹ and R² are independently hydrogen or an alkyl of 1 - 4 carbons, R³ is hydrogen or an alkyl of 1 - 3 carbons or an alkoxy of 1 - 3 carbons.

The active ingredient of the ameliorating composition for dysmnesia, namely pyrazolo[1,5-a]pyridine represented by the above general formula is specifically exemplified by 3-isobutyryl-2-isopropylpyrazolo[1,5-a]pyridine (m.p.: 53.5-54 °C), 2-methyl-3-acetylpyrazolo[1,5-a]pyridine (m.p.: 86-87 °C), 2-ethyl-3-propionylpyrazolo[1,5-a]pyridine (m.p.: 105.5-106 °C), 2-isobutyl-3-isovalerylpyrazolo[1,5-a]pyridine (m.p.: 53-54 °C), 2,6-dimethyl-3-acetylpyrazolo[1,5-a]pyridine (m.p.: 141-142 °C), 2-methyl-3-acetyl-6-methoxypyrazolo[1,5-a]pyridine (m.p.: 123 °C), 3-acetyl-2-isopropylpyrazolo[1,5-a]pyridine (m.p.: 74 °C), 3-acetyl-2-n-propylpyrazolo[1,5-a]pyridine (m.p.: 98-99 °C), 3-formyl-2-isopropylpyrazolo[1,5-a]pyridine (m.p.: 81-81.5 °C), 3-acetylpyrazolo[1,5-a]pyridine (m.p.: 96-97 °C), and 3-isobutyryl-2-methylpyrazolo[1,5-a]pyridine (b.p.: 139-145 °C/5 mmHg).

The dysmnesia ameliorating composition of the present invention may be administered to a patient in the form of an oral composition, a liniment composition, or a composition for injection. Thus the form of the composition is exemplified by tablets, capsules, and granules for oral compositions. It may also be in form of suppositories, ointments, or injections. The other ingredients for various such compositions include, for example, excipients such as starch, lactose, and hydroxypropylcellulose; binders such as hydroxypropylcellulose, and carboxymethylcellulose; lubricants such as magnesium stearate and starch for oral preparations. Color, taste or flavor may be added to oral preparations. Fatty base materials, such as cacao butter and Witepsol (tradename, made by Dynamite Nobel Co., Ltd.), may be used for suppositories. For injections, conventional materials, such as solubilizers, e.g. polyoxyethylene hydrogenated castor oil; isotonicities, e.g. xylitol, and lactose; may be used.

The dysmnesia ameliorating composition of the present invention is administered to a patient in one or three divisional doses of 1 - 500 mg per day, preferably 1 - 200 mg per day, more preferably 1 - 100 mg per day of the active ingredient , namely a pyrazolo[1,5-a]pyridine derivative , the dose being adjusted in accordance with the symptom and the age of she patient. The dysmnesia ameliorating composition of the present invention containing a pyrazolo[1,5-a]pyridine as an active ingredient, exhibits a potent effect in ameliorating dysmnesia in comparison with known agents for the treatment of cerebrovascular disease or agents activating brain metabolism which do not sufficiently ameliorate dysmnesia.

The examples below are intended to illustrate the present invention specifically.

### Example 1

200 mg of 3-isobutyryl-2-isopropylpyrazolo[1,5-a]pyridine (hereinafter referred to as Compound A) were dissolved by heating in 200 ml of physiological saline containing 1 % of polyoxyethylene hydrogenated castor oil to prepare an injection preparation containing Compound A at a concentration of 0.1 %.

Animal experiments were conducted by employing a model of amnesia with the above injection preparation to confirm the dysmnesia ameliorating effect.

### 1. Experimental animals:

Male mice (ddY strain , 5 - 6 weeks of age) were employed.

### 2. Apparatus:

A Passive Avoidance Experiment Box of the step-through type (made by Ohara Ika Sangyo K.K.) consisting of a light (14 x 10 x 11 cm) and a dark (18 x 12.5 x 12 cm) chamber. The light chamber was illuminated with a 20-W fluorescent lamp from approximately 15 cm above the light chamber. The two chambers were separated by a guillotine door. Each chamber had a floor made of a grid of stainless steel. Only the grid of the dark chamber was connected to an electric stimulating apparatus to give an electric shock to the limbs of the animal.

### 3. Acquisition trial:

A mouse was put into the light chamber. After 10 seconds, the guillotine door was opened to allow the mouse to enter the dark chamber. Immediately after the four limbs of the mouse entered the dark room, the guillotine door was closed. Two seconds later, an electric shock of AC 45 - 50 V was given to the mouse for one second, and then immediately the mouse was taken out of the dark room.

### 4. Retention trial:

After 24 hours from the acquisition trial, the mouse was again put into the light chamber, and the same process was repeated except that the electric shock was not given. The time elapsed from the opening of the guillotine door before the mouse entered the dark chamber (Latency) was measured to a maximum of 300 seconds.

### 5. Amnesia-inducing operation:

### (1) Amnesia induction by an electroconvulsive shock:

Immediately after the acquisition trial, an electroconvulsive shock (AC 100 - 140 V, 0.5 seconds) was given to the mouse from the both ears.

### (2) Amnesia induction by cycloheximide:

Thirty minutes before the acquisition trial, cycloheximide was injected subcutaneously at a dose of 120 mg/kg.

### (3) Amnesia induction by carbon dioxide:

Immediately after the acquisition trial, the mouse was put into a container of approximately 300 ml capacity filled with pure carbon dioxide, and was exposed to the gas for 25 seconds.

### 6. Dosage of the test compounds:

The test compounds, nicardipine: a hydrochloride salt of methyl-2-[methyl(phenylmethyl)amino]ethyl ester of 1,4-dihydro-2,6-dimethyl-4-(3-nitrophenyl)-3,5-pyridinedicarboxylic acid; and physostigmine: a methyl carbamate ester of 1,2,3,3a,8,8a-hexahydro-1,3a,8-trimethylpyrrolo[2,3-b]indol-5-ol were dissolved in physiological saline; aniracetam: 1-(4-methoxybenzoyl)-2-pyrrolidinone, and idebenone: 2-(10-hydroxydecyl)-5,6-dimethoxy-3-methyl-2,5-cyclohexadiene-1,4-dione were suspended in physiological saline containing 5 % gum arabicum. The compound was injected intraperitoneally, immediately after the electroconvulsive shock in the test of amnesia induction by electric shock, or 30 minutes before the retention trial in the test of amnesia induction by cycloheximide or carbon dioxide.

### 7. Results:

The results of the experiments are shown in Table 1 to Table 3.

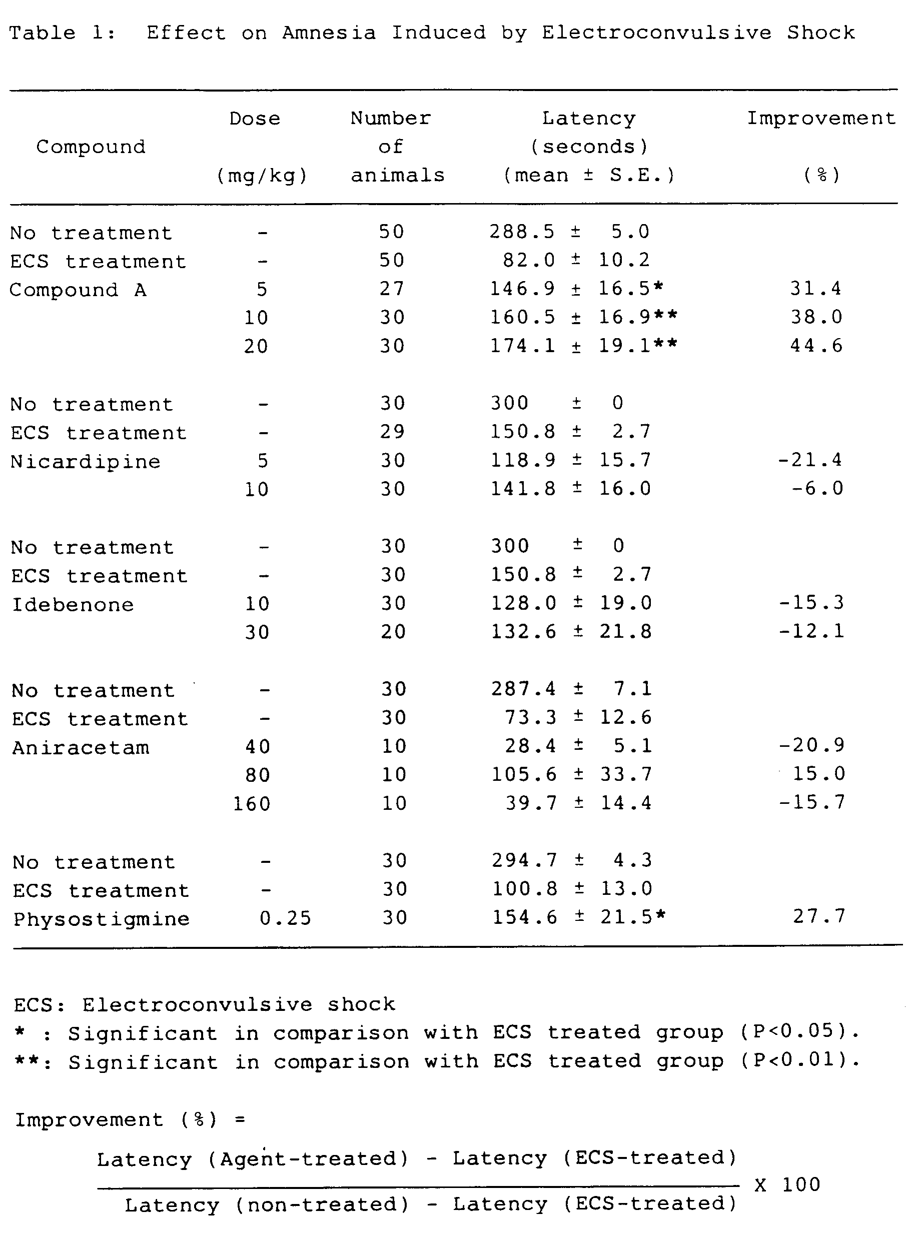

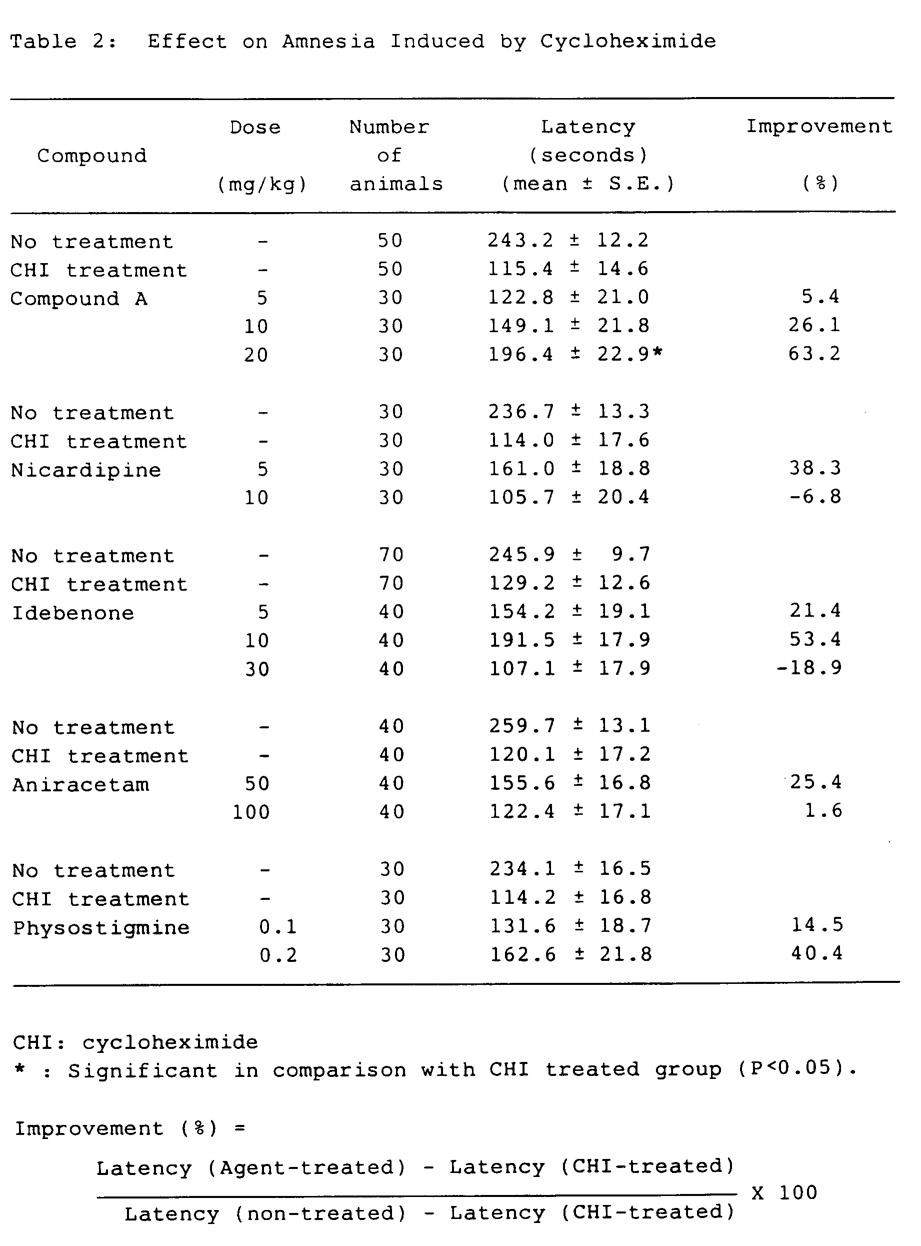

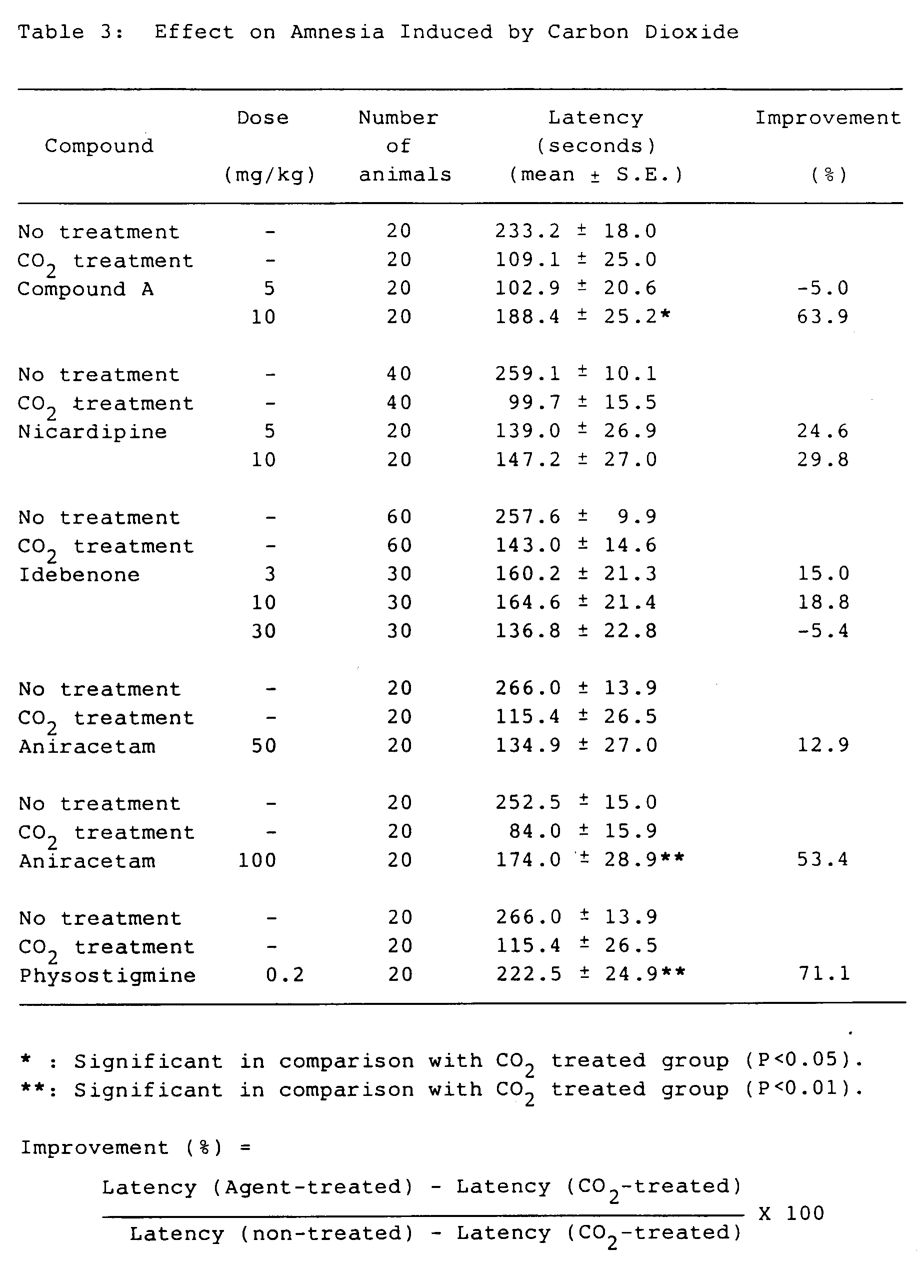

As shown in the above tables, the compound of the present invention exhibits a significant ameliorating effect on experimentally induced amnesia in comparison with an agent ameliorating circulation (nicardipine), an agent activating brain metabolism (idebenone), an intellectualizing agent (aniracetam), and anticholinesterase agent (physostigmine).

### Example 2

20.0 g of Compound A were passed through a 100 mesh sieve, and then mixed with 350.0 g of lactose (150 mesh), 152.5 g of dried starch and 2.5 g of magnesium stearate, and the mixture was passed through a 50 mesh sieve.

After repeated mixing and sieving, the mixture was filled in capsules in a conventional manner to prepare a capsule preparation containing 210 mg of the mixture (8 mg of Compound A) per capsule.

### Example 3

15.0 g of Compound A were passed through a 100 mesh sieve, and then mixed with 244.5 g of lactose (150 mesh), 112.5 g of dried starch and 3.0 g of magnesium stearate, and the mixture was passed through a 50 mesh sieve.

After repeated mixing and sieving, the mixture was filled in capsules in a conventional manner to prepare a capsule preparation containing 250 mg of the mixture (10 mg of Compound A) per capsule.

### Example 4

30.0 g of Compound A were passed through a 100 mesh sieve, and then mixed with 229.5 g of lactose (150 mesh), 112.5 g of dried starch and 3.0 g of magnesium stearate, and the mixture was passed through a 50 mesh sieve.

After repeated mixing and sieving, the mixture was filled in capsules in a conventional manner to prepare a capsule preparation containing 250 mg of the mixture (20 mg of Compound A) per capsule.

### Example 5

Into 24.96 kg of Witepsol W-35 heated to approximately 60 °C and melted, 194 g of Compound A were added and dissolved by stirring The mixture was cooled to approximately 37 °C, and fractionally filled into plastic suppository containers in an amount of 1.3 g per container by means of an automatic suppository filling and packing machine (made by Ramp Co.). Subsequently, having been cooled to 20 - 18 °C, it was sealed and packaged to prepare a suppository containing 10 mg of Compound A per suppository.

## Claims (Claims for the following Contracting State(s): BE, CH, DE, FR, GB, IT, LI, NL, SE)

1. Use of at least one pyrazolo[1,5-a]pyridine derivative represented by the general formula: 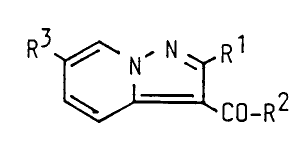 wherein R¹ and R² are independently hydrogen or an alkyl of 1 - 4 carbons, R³ is hydrogen or an alkyl of 1 - 3 carbons or an alkoxy of 1 - 3 carbons for the preparation of a pharmaceutical composition for ameliorating dysmnesia.

## Claims (Claims for the following Contracting State(s): ES)

1. A method for preparing a pharmaceutical composition for ameliorating dysmnesia comprising mixing at least one pyrazolo[1,5-a]-pyridine derivative represented by the general formula: 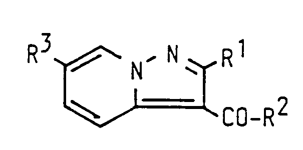 wherein R¹ and R² are independently hydrogen or an alkyl of 1 - 4 carbons, R³ is hydrogen or an alkyl of 1 - 3 carbons or an alkoxy of 1 - 3 carbons with a pharmaceutically acceptable carrier.

## Patentansprüche (Patentansprüche für folgende(n) Vertragsstaat(en): BE, CH, DE, FR, GB, IT, LI, NL, SE)

1. Verwendung mindestens eines Pyrazolo[1,5-a]-pyridinderivates der allgemeinen Formel 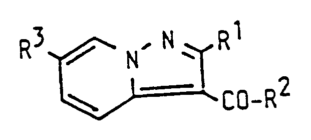 in der R¹ und R² unabhängig voneinander ein Wasserstoffatom oder einen Alkylrest mit 1 bis 4 Kohlenstoffatomen bedeuten, R³ ein Wasserstoffatom, einen Alkylrest mit 1 bis 3 Kohlenstoffatomen oder einen Alkoxyrest mit 1 bis 3 Kohlenstoffatomen bedeutet, zur Herstellung eines Arzneimittels zur Besserung von Dysmnesie.

## Patentansprüche (Patentansprüche für folgende(n) Vertragsstaat(en): ES)

1. Verfahren zur Herstellung eines Arzneimittels zur Besserung von Dysmnesie, umfassend das Mischen mindestens eines Pyrazolo[1,5-a]-pyridinderivates der allgemeinen Formel 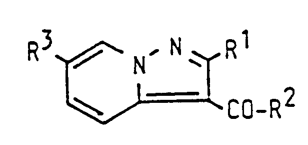 in der R¹ und R² unabhängig voneinander ein Wasserstoffatom oder einen Alkylrest mit 1 bis 4 Kohlenstoffatomen bedeuten, R³ ein Wasserstoffatom, einen Alkylrest mit 1 bis 3 Kohlenstoffatomen oder einen Alkoxyrest mit 1 bis 3 Kohlenstoffatomen bedeutet, mit einem pharmazeutisch verträglichen Träger.

## Revendications (Revendications pour l'(les) Etat(s) contractant(s) suivant(s): BE, CH, DE, FR, GB, IT, LI, NL, SE)

1. Utilisation d'au moins un dérivé de pyrazolo [1,5-a]-pyridine, représenté par la formule générale : 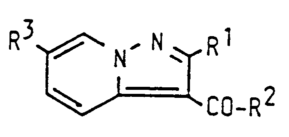 dans laquelle R¹ et R² sont, indépendamment, l'hydrogène ou un alkyle ayant 1-4 atomes de carbone, R³ est l'hydrogène ou un alkyle ayant 1-3 atomes de carbone, ou un alcoxy ayant 1-3 atomes de carbone, pour la préparation d'une composition pharmaceutique pour remédier à la dysmnésie.

## Revendications (Revendications pour l'(les) Etat(s) contractant(s) suivant(s): ES)

1. Procédé pour la préparation d'une composition pharmaceutique pour remédier à la dysmnésie, comprenant le mélange d'au moins un dérivé de pyrazolo [1,5-a]-pyridine, représenté par la formule générale : 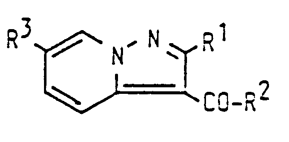 dans laquelle R¹ et R² sont, indépendamment, l'hydrogène ou un alkyle ayant 1-4 atomes de carbone, R³ est l'hydrogène ou un alkyle ayant 1-3 atomes de carbone, ou un alcoxy ayant 1-3 atomes de carbone, avec un véhicule pharmaceutiquement acceptable.
